(19) Europäisches Patentamt
European Patent Office
Office européen des brevets

(11) EP 3 766 960 A1

(12) **EUROPEAN PATENT APPLICATION**
published in accordance with Art. 153(4) EPC

(43) Date of publication:
**20.01.2021 Bulletin 2021/03**

(21) Application number: **20767389.8**

(22) Date of filing: **03.01.2020**

(51) Int Cl.:
***C12N 1/20*** *(2006.01)* ***A23K 10/18*** *(2016.01)*
***B01D 53/84*** *(2006.01)* ***C12R 1/125*** *(2006.01)*

(86) International application number:
**PCT/KR2020/000133**

(87) International publication number:
**WO 2020/179999 (10.09.2020 Gazette 2020/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.03.2019 KR 20190026476**
**02.01.2020 KR 20200000489**

(71) Applicant: **CJ Cheiljedang Corporation**
**Seoul 04560 (KR)**

(72) Inventors:
- **KIM, Yu Jin**
  **Suwon-si, Gyeonggi-do 16509 (KR)**
- **PARK, Min Ah**
  **Suwon-si, Gyeonggi-do 16459 (KR)**
- **OH, Eun Seon**
  **Seongnam-si, Gyeonggi-do 13462 (KR)**
- **WOO, Seo Hyung**
  **Suwon-si, Gyeonggi-do 16519 (KR)**

(74) Representative: **J A Kemp LLP**
**14 South Square**
**Gray's Inn**
**London WC1R 5JJ (GB)**

(54) **BACILLUS SUBTILIS CJBS303 AND COMPOSITION COMPRISING SAME**

(57) Provided are a *Bacillus subtilis* CJBS303 strain and use thereof. Also, provided are a strain having an effect of reducing livestock manure-derived odors, a composition and a microbial preparation, each including the strain, and a feed composition including the composition.

FIG. 2

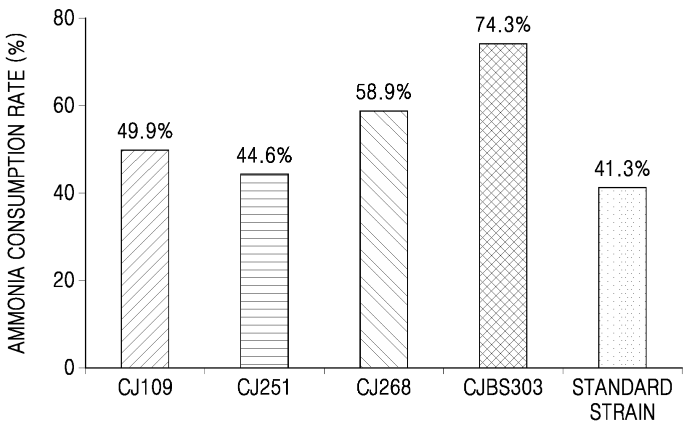


EP 3 766 960 A1

<110> CJ CheilJedang Corporation

<120> Bacillus subtilis CJBS303 and composition comprising the same

<130> PX060611PCT

<150> KR 10-2019-0026476
<151> 2019-03-07

<150> KR 10-2020-0000489
<151> 2020-01-02

<160> 1

<170> KoPatentIn 3.0

<210> 1
<211> 1479
<212> DNA
<213> 16s rDNA

<400> 1

```
cttcacccca atcatctgtc ccaccttcgg cggctggctc ctaaaaggtt acctcaccga         60

cttcgggtgt tacaaactct cgtggtgtga cgggcggtgt gtacaaggcc cgggaacgta        120

ttcaccgcgg catgctgatc cgcgattact agcgattcca gcttcacgca gtcgagttgc        180

agactgcgat ccgaactgag aacagatttg tgggattggc ttaacctcgc ggtttcgctg        240

ccctttgttc tgtccattgt agcacgtgtg tagcccaggt cataaggggc atgatgattt        300

gacgtcatcc ccaccttcct ccggtttgtc accggcagtc accttagagt gcccaactga        360

atgctggcaa ctaagatcaa gggttgcgct cgttgcggga cttaacccaa catctcacga        420

cacgagctga cgacaaccat gcaccacctg tcactctgcc cccgaagggg acgtcctatc        480

tctaggattg tcagaggatg tcaagacctg gtaaggttct tcgcgttgct tcgaattaaa        540

ccacatgctc caccgcttgt gcgggccccc gtcaattcct ttgagtttca gtcttgcgac        600

cgtactcccc aggcggagtg cttaatgcgt tagctgcagc actaaggggc ggaaaccccc        660

taacacttag cactcatcgt ttacggcgtg gactaccagg gtatctaatc ctgttcgctc        720

cccacgcttt cgctcctcag cgtcagttac agaccagaga gtcgccttcg ccactggtgt        780

tcctccacat ctctacgcat ttcaccgcta cacgtggaat tccactctcc tcttctgcac        840

tcaagttccc cagtttccaa tgaccctccc cggttgagcc gggggctttc acatcagact        900

taagaaaccg cctgcgagcc ctttacgccc aataattccg gacaacgctt gccacctacg        960

tattaccgcg gctgctggca cgtagttagc cgtggctttc tggttaggta ccgtcaaggt       1020

gccgccctat ttgaacggca cttgttcttc cctaacaaca gagctttacg atccgaaaac       1080

cttcatcact cacgcggcgt tgctccgtca gactttcgtc cattgcggaa gattccctac       1140

tgctgcctcc cgtaggagtc tgggccgtgt ctcagtccca gtgtggccga tcaccctctc       1200

aggtcggcta cgcatcgtcg ccttggtgag ccgttacctc accaactagc taatgcgccg       1260
```

```
cgggtccatc tgtaagtggt agccgaagcc accttttatg tctgaaccat gcggttcaaa      1320
caagcatccg gtattagccc cggtttcccg gagttatccc agtcttacag gcaggttacc      1380
cacgtgttac tcacccgtcc gccgctaaca tcagggagca agctcccatc tgtccgctcg      1440
acttgcatgt attaggcacg ccgccagcgt tcgtcctga                              1479
```

## Description

TECHNICAL FIELD

The present disclosure relates to *Bacillus subtilis* CJBS303 having an effect of reducing livestock manure-derived odors, and a composition including the same.

BACKGROUND ART

Recently, odors generated in livestock farms have become a major social problem resulting in an increase in the incidence of complaints in surrounding villages and the occurrence of diseases, as well as environmental pollution. The main cause of odors in livestock farms is gases produced by anaerobic fermentation during production of livestock manure and storage and handling of livestock manure. Accordingly, the Korea Ministry of Environment and the Korea Pork Producers Association designated 12 odorous compounds, including ammonia, methyl mercaptan, hydrogen sulfide, etc., and designated the final 22 odorous compounds and established emission standards in 2010. In addition, the Korea Pork Producers Association proposed odor management standards for livestock farms, based on the results of analyzing 133 farms for two years from 2015 to 2016, and as one of the standards, a dilution factor of complex odor in the farm is required to be managed at 1000-fold or less. Here, complex odor refers to odor that stimulates a person's sense of smell and gives discomfort and disgust while two or more odor compounds are present in combination.

Ammonia ($NH_3$), which is one of the typical harmful gases and odor compounds in a pig pen, is a colorless, irritating, circulating gas that is detectable even at relatively low concentrations. Ammonia is known to be produced during the process of deamination of amino acids. Another source of ammonia includes urea and nitric acid. Ammonia gas irritates wet body tissues, causing workers' eyes to sting and causing congestion of pigs' eyes. According to research data on swine productivity depending on ammonia concentrations in a pig pen, when ammonia is over 50 ppm, about 12% of growth retardation occurs in piglets, and when ammonia is at 100 ppm to 150 ppm, 30% of pigs show growth inhibition and symptoms of respiratory diseases.

Other major odorous gases include volatile sulfur compounds, including hydrogen sulfide ($H_2S$), methyl mercaptan ($CH_3SH$), etc. These volatile sulfur compounds are produced by reduction of sulfates and metabolism of amino acids containing sulfur. Hydrogen sulfide is a toxic gas, and is heavier than air, and thus settles to the surface of slurry in a pig pen. Hydrogen sulfide causes headaches, dizziness and nausea in workers, and causes loss of appetite and respiratory diseases in pigs. In addition, odorous gases of livestock farms include indoles and phenols such as indole, skatole, p-cresol, etc., and volatile fatty acids (VOCs) such as propionic acid, butyric acid, acetic acid, etc. There is a need to develop a technology capable of reducing generation of these odorous gases.

In view of this technical background, there have been many efforts to reduce odors generated in livestock farms, and as a result, a strain of the genus *Bacillus* capable of remarkably reducing odorous gases such as ammonia, hydrogen sulfide, etc. derived from livestock manure was isolated and identified. Based on this, the present disclosure has been completed.

[Prior Art Document]

[Patent Document]

(Patent Document 1) Korean Patent No. 10-1229865

DESCRIPTION OF EMBODIMENTS

TECHNICAL PROBLEM

An object of the present disclosure is to provide a *Bacillus subtilis* CJBS303 strain deposited with the Accession No. KCCM12435P.

Another object of the present disclosure is to provide a composition including the *Bacillus subtilis* CJBS303 strain, a culture of the strain, a concentrate of the culture, or a dry product of the concentrate.

Still another object of the present disclosure is to provide a feed composition including the composition.

Still another object of the present disclosure is to provide a microbial preparation for removing livestock odors, the microbial preparation including the *Bacillus subtilis* CJBS303 strain.

Still another object of the present disclosure is to provide a method of reducing odors from manure of an individual, the method including administering, to the individual, the *Bacillus subtilis* CJBS303 strain, the composition, or the microbial preparation.

Still another object of the present disclosure is to provide a method of preparing the microbial preparation, the method including culturing the *Bacillus subtilis* CJBS303 strain deposited with the Accession No. KCCM12435P; and mixing the recovered strain with an additive.

Other objects and advantages of the present disclosure will become more fully apparent from the following detailed description of the embodiments, the appended claims and the accompanying drawings. Since contents that are not described in the present specification may be sufficiently recognized and inferred by those skilled in the art or similar art, a description thereof will be omitted.

SOLUTION TO PROBLEM

Each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

An aspect of the present disclosure provides a *Bacillus subtilis* CJBS303 strain deposited with the Accession No. KCCM12435P.

The strain may have an effect of reducing livestock manure-derived odors. For example, the strain may reduce a level of at least one odorous gas selected from the group consisting of ammonia, hydrogen sulfide, methyl mercaptan, indoles, phenols, and volatile fatty acids (VOCs).

As used herein, the term "livestock manure-derived odors" may be used interchangeably with "odors derived from livestock manure" or "odors derived from domestic animal manure". The odors may be generated from odorous gases derived from livestock manure, for example, odorous gases by anaerobic fermentation during treatment of livestock manure.

According to one exemplary embodiment, a CJBS303 strain with the highest ammonia consumption rate was selected from a total of 456 strains which were isolated from samples such as soybean paste, red pepper paste, blocks of fermented soybeans, etc., which are traditional Korean sauces. Sequence analysis of 16s rDNA of the selected strain was performed to identify a nucleotide sequence of SEQ ID NO: 1, which shows 99% homology with *Bacillus subtilis*. Therefore, the strain was named *Bacillus subtilis* CJBS303, and the *Bacillus subtilis* CJBS303 strain was deposited in the Korean Culture Center of Microorganisms on February 13, 2019, with the Accession No. KCCM12435P.

In one specific embodiment, the *Bacillus subtilis* CJBS303 strain may reduce levels of odorous gases derived from livestock manure. The odorous gases may include ammonia; hydrogen sulfide; methyl mercaptan; indoles and phenols such as indole, skatole, p-cresol, etc.; and volatile fatty acids (VOCs) such as propionic acid, butyric acid, acetic acid, etc., and may be, for example, at least one odorous gas selected from the group consisting of ammonia, hydrogen sulfide, methyl mercaptan, indoles, phenols, volatile fatty acids.

According to one exemplary embodiment, it was confirmed that addition of the *Bacillus subtilis* CJBS303 strain reduced levels of ammonia derived from a pig fecal slurry, and feeding of the strain also reduced levels of ammonia and hydrogen sulfide derived from manure. Therefore, the present disclosure may provide a novel strain having an effect of reducing odors derived from livestock manure.

Another aspect of the present disclosure provides a composition including the *Bacillus subtilis* CJBS303 strain, a culture of the strain, a concentrate of the culture, or a dry product of the concentrate.

The *Bacillus subtilis* CJBS303 strain is the same as described above.

In one specific embodiment, the culture of the *Bacillus subtilis* CJBS303 strain may be prepared by seeding the strain in a medium for microbial culture according to a method of culturing a microorganism known in the art (e.g., stationary culture, agitating culture). Further, the concentrate of the strain culture and the dry product thereof may also be easily prepared according to a method of treating, concentrating, or drying a microorganism or a culture, which is known in the art.

According to one exemplary embodiment, the feeding of the *Bacillus subtilis* CJBS303 strain may reduce levels of ammonia and hydrogen sulfide derived from manure, thereby contributing to improvement of the livestock environment and improvement of livestock productivity. Accordingly, the *Bacillus subtilis* CJBS303 strain, the culture of the strain, the concentrate of the culture, or the dry product of the concentrate may be included in the composition, thereby being applied as a feed additive.

The composition of the present disclosure may be prepared in the form of a powder or granules, and as needed, the composition may further include any one or one or more of organic acids such as citric acid, humalic acid, adipic acid, lactic acid, malic acid, etc., phosphates such as sodium phosphate, potassium phosphate, acidic pyrophosphates, polyphosphates, etc., and natural antioxidants such as polyphenols, catechins, alpha-tocopherols, rosemary extracts, vitamin C, green tea extracts, licorice extracts, chitosan, tannic acid, phytic acid, etc.

The composition of the present disclosure may further include crops, for example, crushed or shredded wheat, oats, barley, corn, and rice; vegetable protein feeds, for example, feeds mainly consisting of rape, soybean, and sunflower; animal protein feeds, for example, blood meal, meat meal, bone meal, and fish meal; and sugar and dairy products, for example, various dry ingredients consisting of milk powder and whey powder, and may further include nutritional supplements, digestibility- and absorption-promoters, growth promoters, etc.

The composition of the present disclosure may be administered to animals individually or in combination with other additives selected from edible carriers. Further, the composition may be topdressing, may be directly mixed with feeds or may be easily administered to animals as oral dosage forms separately from animal feeds. When administered separately from feeds, the composition may be combined with pharmaceutically acceptable edible carriers and prepared into immediate-release formulations or sustained-release formulations, as well known in the art. The edible carriers may be a solid or a liquid, for example, corn starch, lactose, sucrose, soy flake, peanut oil, olive oil, sesame oil, and propylene glycol. When solid carriers are used, the composition may be in the form of a tablet, a capsule, a powder, a troche, or a lozenge, or may be a not-dispersed topdressing. When liquid carriers are used, the composition may be a formulation of soft gelatin capsule, syrup, suspension, emulsion, or solution.

The composition of the present disclosure may include, for example, a preservative, a stabilizer, a wetting agent, an emulsifier, a liquefying agent, a cryoprotectant, an excipient, etc. The cryoprotectant may be one or more selected from the group consisting of glycerol, trehalose, maltodextrin, skim milk, and starch.

The preservative, the stabilizer, or the excipient may be included in an effective amount sufficient to reduce deterioration of the *Bacillus subtilis* CJBS303 strain which is included in the composition. Further, the cryoprotectant may be included in the composition in an effective amount sufficient to reduce deterioration of the *Bacillus subtilis* CJBS303 strain which is included in the composition, when the composition is in a dry state.

The composition may be added to animal feeds by means of dipping, spraying or mixing for use.

The composition of the present disclosure may be applied to a diet for various animals such as mammals and poultry. The mammals may include pigs, cows, sheep, goats, laboratory rodents, pets (e.g., dogs, cats), etc., and the poultry may include chickens, turkeys, ducks, geese, pheasant, quail, etc.

Still another aspect of the present disclosure provides a feed composition including the composition.

The feed composition of the present disclosure may be formulated in a common feed form, and may include common feed ingredients.

As used herein, the term "feed composition" refers to a feed that is fed to animals. The feed composition refers to a material that supplies organic or inorganic nutrients needed to maintain animals' life or to produce meat or milk. The feed composition may further include nutrients needed to maintain animals' life or to produce meat or milk.

The content of the composition including the *Bacillus subtilis* CJBS303 strain, the culture of the strain, the concentrate of the culture, or the dry product of the concentrate included in the feed composition of the present disclosure may be appropriately adjusted according to the type and age of the livestock, the type of application, the desired effect, etc., and the composition may be used, for example, in an amount of 0.01%(w/w) to 1%(w/w), 0.01%(w/w) to 0.5 %(w/w), or 0.15%(w/w) to 0.5 %(w/w).

In one specific embodiment, the feed composition of the present disclosure may include the *Bacillus subtilis* CJBS303 strain in an amount of $1.0\times10^7$ cfu/kg to $1.0\times10^{11}$ cfu/kg, based on the total weight of the composition. The content of the strain may be $1.0\times10^7$ cfu/kg to $5.0\times10^{10}$ cfu/kg, $1.0\times10^7$ cfu/kg to $1.0\times10^{10}$ cfu/kg, $1.0\times10^7$ cfu/kg to $5.0\times10^9$ cfu/kg, $1.0\times10^7$ cfu/kg to $1.0\times10^9$ cfu/kg, $1.0\times10^7$ cfu/kg to $5.0\times10^8$ cfu/kg, $1.0\times10^7$ cfu/kg to $1.0\times10^8$ cfu/kg, $1.0\times10^7$ cfu/kg to $5.0\times10^7$ cfu/kg, $1.0\times10^8$ cfu/kg to $5.0\times10^{10}$ cfu/kg, $1.0\times10^8$ cfu/kg to $1.0\times10^{10}$ cfu/kg, $1.0\times10^8$ cfu/kg to $5.0\times10^9$ cfu/kg, $1.0\times10^8$ cfu/kg to $1.0\times10^9$ cfu/kg, $1.0\times10^8$ cfu/kg to $5.0\times10^8$ cfu/kg, $1.0\times10^9$ cfu/kg to $5.0\times10^{10}$ cfu/kg, $1.0\times10^9$ cfu/kg to $1.0\times10^{10}$ cfu/kg, or $1.0\times10^9$ cfu/kg to $5.0\times10^9$ cfu/kg, but may be appropriately adjusted according to the type and age of the livestock, the type of application, the desired effect, etc.

The feed composition of the present disclosure may be mixed, for administration, with one or more of organic acids such as citric acid, fumaric acid, adipic acid, lactic acid, etc.; phosphates such as potassium phosphate, sodium phosphate, polymerized phosphate, etc.; natural antioxidants such as polyphenols, catechins, tocopherols, vitamin C, green tea extract, chitosan, tannic acid, etc., and as needed, other common additives such as an anti-influenza agent, a buffer, a bacteriostatic agent, etc. may be added. Further, the feed composition of the present disclosure may also be formulated into injectable formulations such as an aqueous solution, a suspension, an emulsion,

etc., capsules, granules, or tablets by additionally adding a diluent, a dispersant, a surfactant, a binder, or a lubricant thereto. Further, the feed composition of the present disclosure may include various kinds of auxiliary substances such as amino acids, inorganic salts, vitamins, antioxidants, antifungal agents, antimicrobial agents, etc., main ingredients such as plant-based protein feeds such as pulverized or crushed wheat, barley, corn, etc., animal-based protein feeds such as blood meal, meat meal, fish meal, etc., animal fat and vegetable oil, as well as nutritional supplements, growth promoters, digestibility promoters, and a prophylactic agent.

The feed composition of the present disclosure may be mixed in an amount of about 10 g to about 500 g per 1 kg, for example, about 10 g to about 100 g per 1 kg, based on the dry weight of a feed for livestock. After completely mixing, the feed composition may be provided to a mesh or may be subjected to a pelletization, expansion, or extrusion process through additional processes.

Still another aspect of the present disclosure provides a microbial preparation for removing livestock odors, the microbial preparation including the *Bacillus subtilis* CJBS303 strain.

According to one exemplary embodiment, feeding of the *Bacillus subtilis* CJBS303 strain may reduce levels of ammonia and hydrogen sulfide derived from manure, and may significantly reduce levels of ammonia, hydrogen sulfide, methyl mercaptan, volatile organic compounds, and complex odor in a pen. Therefore, the strain of the present disclosure may be applied as a microbial preparation for removing livestock odors.

In one specific embodiment, the microbial preparation of the present disclosure may reduce levels of at least one odorous gas selected from the group consisting of ammonia, hydrogen sulfide, methyl mercaptan, volatile fatty acids, and combinations thereof.

In one specific embodiment, the microbial preparation of the present disclosure may be in the form of a solid or a liquid prepared according to a method known in the art. When the microbial preparation of the present disclosure is, for example, in the form of a solid, the strain is attached to a carrier, and then dried to the water content of 0.1% by weight to 10% by weight, based on the total weight of the microbial preparation, and commercialized in the form of beads or in the form of powder by pulverization. As the carrier, one or more selected from the group consisting of powdered clays, activated carbon, coke, volcanic ashes, and combustion ashes may be used, and the clays may be one or more selected from the group consisting of zeolite, vermiculite, diatomite, kaolin, potter's clay, feldspar, and talc, but is not limited thereto. Further, an excipient may be added to a solid form of the microbial preparation. As the excipient, amino acids, vitamin C, vitamin E, chitosan, and glucose may be used alone or in combination of two or more thereof, but is not limited thereto.

When the microbial preparation of the present disclosure is, for example, a liquid form, it may be prepared by mixing the culture of the strain and mixing glucose or glycerin to stabilize the microorganism. The microbial preparation may be prepared by using the culture at a final concentration of 5% by weight to 40% by weight, for example, at a final concentration of 10% by weight to 20% by weight, based on the total weight of the microbial preparation, but is not limited thereto.

Still another aspect of the present disclosure provides a method of reducing odors from manure of an individual, the method including administering, to the individual, the *Bacillus subtilis* CJBS303 strain, the composition, or the microbial preparation.

As used herein, the term "individual" refers to a subject, of which manure odors are intended to reduce, and more specifically, a non-human animal. For example, the non-human animal may include mammals and poultry, and the mammals may include pigs, cows, sheep, goats, dogs, cats, etc., and may include laboratory rodents, pets, etc., and the poultry may include chickens, turkeys, ducks, geese, pheasant, quail, etc., but is not limited thereto.

Still another aspect of the present disclosure provides a method of preparing the microbial preparation, the method including culturing the *Bacillus subtilis* CJBS303 strain deposited with the Accession No. KCCM12435P; and mixing the recovered strain with an additive.

Culturing of the strain may be performed according to a method known in the art, and the culturing method may include one or more selected from the group consisting of batch, continuous, and fed-batch cultures.

A medium used for the culturing may be a medium that may satisfy the requirements of a specific microorganism. The medium may be a medium selected from the group consisting of carbon sources, nitrogen sources, trace elements, and combinations thereof.

The carbon source may be a carbon source selected from the group consisting of carbohydrates, fats, fatty acids, alcohols, organic acids, and combinations thereof. The carbohydrate may be glucose, sucrose, lactose, fructose, maltose, starch, cellulose, or a combination thereof. The fat may be soybean oil, sunflower oil, castor oil, coconut oil, or a combination thereof. The fatty acid may be palmitic acid, stearic acid, linoleic acid, or a combination thereof. The alcohol may be glycerol or ethanol. The organic acid may include acetic acid.

The nitrogen source may include an organic nitrogen source, an inorganic nitrogen source, or a combination thereof. The organic nitrogen source may be selected from the group consisting of peptone, yeast extract, beef stock, malt extract, corn steep liquor (CSL), soybean wheat, and combinations thereof. The inorganic nitrogen source may be selected from the group consisting of urea, ammonium sulfate, ammonium chloride, ammonium phosphate, ammonium carbonate, ammonium nitrate and combinations thereof.

The medium may include those selected from the group consisting of phosphorus, metal salts, amino acids, vitamins, precursors, and combinations thereof. The source of phosphorus may include potassium dihydrogen phosphate, dipotassium hydrogen phosphate, or a sodium-containing salt corresponding thereto. The metal salt may be magnesium sulfate or iron sulfate.

The medium or individual components constituting the medium may be added in a batch, continuous, or fed-batch mode.

The method of preparing the microbial preparation may further include recovering the strain from the culture in which the strain has been cultured, after culturing the strain. The recovering of the strain may be performed by a method known in the art, for example, by a method of recovering precipitates generated after centrifuging the culture.

In the method of preparing the microbial preparation, the additive may be a cryoprotectant, and the method may further include freeze-drying the obtained mixture, after mixing the recovered strain with the additive. The freeze-drying may be performed by a method known in the art.

The strain in the freeze-dried microbial preparation which is obtained in freeze-drying may be in a viable state. In addition, the freeze-dried microbial preparation may include the cryoprotectant in an effective amount sufficient to reduce deterioration of the strain in the agent, and thus deterioration of the strain in the viable state may be reduced.

In the method of preparing the microbial preparation, the microbial preparation may include, based on the total weight of the microbial preparation, the *Bacillus subtilis* CJBS303 strain of $1.0\times10^7$ cfu/kg to $1.0\times10^{11}$ cfu/kg, for example, the *Bacillus subtilis* CJBS303 strain of $1.0\times10^7$ cfu/kg to $5.0\times10^{10}$ cfu/kg, $1.0\times10^7$ cfu/kg to $1.0\times10^{10}$ cfu/kg, $1.0\times10^7$ cfu/kg to $5.0\times10^9$ cfu/kg, $1.0\times10^7$ cfu/kg to $1.0\times10^9$ cfu/kg, $1.0\times10^7$ cfu/kg to $5.0\times10^8$ cfu/kg, $1.0\times10^7$ cfu/kg to $1.0\times10^8$ cfu/kg, $1.0\times10^7$ cfu/kg to $5.0\times10^7$ cfu/kg, $1.0\times10^8$ cfu/kg to $5.0\times10^{10}$ cfu/kg, $1.0\times10^8$ cfu/kg to $1.0\times10^{10}$ cfu/kg, $1.0\times10^8$ cfu/kg to $5.0\times10^9$ cfu/kg, $1.0\times10^8$ cfu/kg to $1.0\times10^9$ cfu/kg, $1.0\times10^8$ cfu/kg to $5.0\times10^8$ cfu/kg, $1.0\times10^9$ cfu/kg to $5.0\times10^{10}$ cfu/kg, $1.0\times10^9$ cfu/kg to $1.0\times10^{10}$ cfu/kg, or $1.0\times10^9$ cfu/kg to $5.0\times10^9$ cfu/kg, which may be appropriately adjusted according to the type and age of the livestock to be applied, the type of application, the desired effect, etc.

## ADVANTAGEOUS EFFECTS OF DISCLOSURE

A *Bacillus subtilis* CJBS303 strain according to the present disclosure may effectively reduce generation of odorous gases from livestock manure or livestock housing.

Accordingly, a composition and a microbial preparation, each including the strain, may contribute to improvement of the livestock environment and improvement of livestock productivity through reduction of odors.

## BRIEF DESCRIPTION OF DRAWINGS

FIG. 1 shows results of evaluating ammonia consumption capacity according to one exemplary embodiment, wherein color development of culture media according to ammonia consumption was visually examined;

FIG. 2 shows results of quantitatively comparing ammonia consumption rates of CJ109, CJ251, CJ268, and CJBS303 strains which were cultured in ammonia-containing media;

FIG. 3 shows electron microscopy results of examining morphological characteristics of the CJBS303 strain;

FIG. 4 shows results of visually examining hemolysis of the CJBS303 strain;

FIG. 5 shows results of examining changes of ammonia generation from pig fecal slurries according to addition of the CJBS303 strain;

FIG. 6 shows results of examining changes in ammonia and hydrogen sulfide generation from manure according to feeding of the CJBS303 strain;

FIGS. 7A to 7E show effects of reducing odorous gases in a pig pen according to feeding of the CJBS303 strain, wherein FIGS. 7A, 7B, 7C, 7D, and 7E show results of examining complex odor levels in a pig pen, ammonia levels in the pig pen, hydrogen sulfide levels in the pig pen, volatile fatty acid (VOCs) levels in the pig pen, and methyl mercaptan levels in the pig pen over time, respectively; and

FIGS. 8A to 8D show effects of reducing odorous gases in a pig pen according to feeding of the CJBS303 strain, wherein FIGS. 8A, 8B, 8C, and 8D show results of comparing ammonia levels in a pig pen, hydrogen sulfide levels in the pig pen, methyl mercaptan levels in the pig pen, and complex odor levels in the pig pen, respectively.

## MODE OF DISCLOSURE

Hereinafter, the present disclosure will be described in more detail with reference to exemplary embodiments. However, these exemplary embodiments are only for illustrating the present disclosure, and the scope of the present disclosure is not limited to these exemplary embodiments.

### Example 1. Isolation and Identification of Strain Having Effect of Reducing Livestock Manure-Derived Odors

#### (1) Acquisition of samples and Isolation of strain

Samples such as soybean paste, red pepper paste, blocks of fermented soybeans, etc., which are traditional Korean sauces, were obtained, and the samples were serially diluted and then plated on a Brain Heart Infusion (BHI, Difco) solid medium containing 3% sodium chloride, followed by incubation at 37°C for 24 hr. Strains isolated from each sample were grouped and separated according to colonies. The selected colonies were purified by transferring the colonies to a new medium over three times, and a total of 465 strains purified and cultured were stored in a medium, to which 20% glycerol was added, at a temperature below -70°C.

(2) Selection of strain by evaluation of ammonia consumption capacity

To select strains that consume ammonia which is a main cause of odors, ammonia consumption capacity of the strains was evaluated. The ammonia consumption capacity was examined by a qualitative method of using a reagent (sulfanilic acid, N, N-Dimethyl-1-naphthylamine) that develops a color by reacting with nitrite resulting from oxidation of ammonia. In detail, ammonia-added broths (containing 4.95 g/L of $(NH_4)_2SO_4$, 8.82 g/L of $K_2HPO_4$, 1.1 ml/L of 1 M $MgSO_4$ solution, 0.3 ml/L of 1 M $CaCl_2$ solution, 0.5 ml/L of 30 mM $FeSO_4$ solution, 0.04 ml/L of 50 mM $CuSO_4$ solution, 0.7 g/L of $NaH_2PO_4$, and 12 ml/L of 5%(W/V) $Na_2CO_3$ anhydrous) were prepared, and then each of the strains was seeded at a concentration of 0.01% in 10 ml of the prepared broth, followed by incubation at 30°C for 14 days. Thereafter, each of the culture broths was centrifuged, and 1 ml of each supernatant was collected. 100 $\mu$l of the reagent that develops a color by reacting with nitrite was added thereto. Each culture supernatant, to which the reagent was added, was allowed to react at 25°C for 10 min to induce color development, and then color development of the culture broth was observed. Meanwhile, in the following exemplary embodiments, *Bacillus subtilis* KCCM11143P disclosed in Korean Patent Publication No. 10-2012-0088436 was used as a standard strain.

As shown in FIG. 1, when nitrite is produced by consumption or oxidation of ammonia, the culture broth developed dark purple, and strains having ammonia consumption capacity were primarily selected, based on the color development. As a result, of 456 kinds of strains isolated from the samples, 4 kinds of strains having significant ammonia consumption capacity, specifically, CJ109, CJ251, CJ268, and CJBS303 were selected.

In addition, the ammonia consumption was quantitatively compared between 4 kinds of the strains primarily selected. Specifically, after seeding each of the strains at a concentration of 0.1% in a BHI liquid medium, the strain was incubated at 200 rpm and 37°C for 15 hr to activate the strain. Each of the activated strains was seeded at a concentration of 1% in an ammonia-containing medium (containing 0.5 g/L of $(NH_4)_2SO_4$, 13.5 g/L of $NaH_2PO_4$, 0.7 g/L of $K_2HPO_4$, 0.1 g/L of $MgSO_4 \cdot 7H_2O$, 0.18 g/L of $CaCl_2 \cdot 2H_2O$, 0.5 g/L of NaHCOs, 0.014 g/L of $FeCl_3 \cdot 6H_2O$, 0.5 g/L of glucose), followed by incubation at 200 rpm and 37°C for 6 hr. After the culture was completed, the culture broth was centrifuged, from which only the culture supernatant was recovered. The ammonia content in the initial medium and the residual amount of ammonia in the recovered culture supernatant were quantified, and ammonia consumption rates were calculated according to Equation 1.

[Equation 1]

$$\text{Ammonia consumption rate (\%)} = (A-B / A) \times 100$$

(A: initial ammonia content, B: ammonia content after completing culture)

As a result, as shown in FIG. 2, among 4 kinds of the strains primarily selected, the CJBS303 strain showed the highest ammonia consumption rate of 74.3%. Therefore, CJBS303, which has the highest ammonia consumption rate, was finally selected as a strain having an effect of reducing odors derived from livestock manure.

(3) Morphological and biochemical characterization

Morphological and biochemical characteristics of the CJBS303 strain were analyzed. Specifically, morphological characteristics of the strain were analyzed by Gram staining and electron microscopy, and the biochemical characteristics of the strain were analyzed by examining sugar fermentation patterns of the strain through an API 50 CHB system (biomerieux Vitek, Inc., France).

As a result, it was confirmed that the CJBS303 strain is a Gram-positive, rod-shaped bacterium, as shown in FIG. 3. As shown in Table 1, the CJBS303 strain was confirmed to exhibit sugar fermentation patterns similar to *Bacillus subtilis.*

[Table 1]

| Sample | Fermentation | Sample | Fermentation |
|---|---|---|---|
| Control | - | Esculine | + |
| Glycerol | + | Salicine | + |

(continued)

| Sample | Fermentation | Sample | Fermentation |
|---|---|---|---|
| Erythritol | - | Cellobiose | + |
| D-Arabinose | - | Maltose | + |
| L-Arabinose | - | Lactose | + |
| Ribose | + | Melibiose | - |
| D-Xylose | + | Saccharose | + |
| L-Xylose | - | Trehalose | + |
| Adonitol | - | Inuline | - |
| β Methyl-xyloside | - | Melezitose | - |
| Galactose | - | D-Raffinose | + |
| D-Glucose | + | Amidon | - |
| D-Fructose | + | Glycogene | - |
| D-Mannose | + | Xylitol | - |
| L-Sorbose | - | β Gentiobiose | - |
| Rhamnose | - | D-Turanose | - |
| Dulcitol | - | D-Lyxose | - |
| Inositol | + | D-Tagatose | - |
| Mannitol | + | D-Fucose | - |
| Sorbitol | + | L-Fucose | - |
| α Methyl-D-mannoside | - | D-Arabitol | - |
| α Methyl-glucoside | + | L-Arabitol | - |
| *N* Acetyl glucosamine | - | Gluconate | - |
| Amygdaline | + | 2 keto-gluconate | - |
| Arbutine | + | 5 keto-gluconate | - |

(4) Identification of Strain

For identification of the strain, molecular phylogenetic analysis of a DNA sequence was performed. To this end, the gene of 16s rDNA was amplified using PCR premix (Bioneer, Korea) and universal primers of 27F (5' AGAGTTTGATCMTGGCTCAG 3'), and 1492R (5' GGTTACCTTGTTACGACTT 3'). When the gene was amplified, the total reaction solution was set to 20 μl, and repeated 30 times in total under conditions of at 94°C for 1 min, at 56°C for 1 min, and at 72°C for 1 min. The 16s rDNA sequence of CJBS303 strain was the same as SEQ ID NO: 1, which showed 99% homology with *Bacillus subtilis.* In the following exemplary embodiments, the isolated strain was named '*Bacillus subtilis* CJBS303' or 'CJBS303 strain' (Accession No. KCCM12435P).

Example 2. Evaluation of Safety of CJBS303 Strain

To confirm safety of the CJBS303 strain, hemolysis of the strain was evaluated. In detail, the CJBS303 strain was streaked on a blood agar plate (5% sheep blood, Hanil-KOMED, Korea), and then incubated at 37°C for 24 hr. Thereafter, hemolysis on the cultured medium was visually observed.

As a result, as shown in FIG. 4, the CJBS303 strain showed no hemolysis, indicating safety of *Bacillus subtilis* CJBS303 strain.

Example 3. Evaluation of Effect of Reducing Livestock Manure Odor of CJBS303 Strain

(1) Effect of reducing odorous gases in fecal slurry

The effect of reducing odorous gases in a pig fecal slurry of the CJBS303 strain was compared with that of the standard strain. In detail, after culturing the CJBS303 strain in a trypticase soy broth (TSB), 4 ml of a solution containing $1X10^8$ cfu/kg of the CJBS303 strain was added to 400 ml of pig manure slurry, which was incubated under shaking at 120 rpm and 39°C under facultative aerobic conditions for a total of 48 hr. 24 hr and 48 hr from the time of incubation, the amount of ammonia gas was measured for a total of twice using a portable odorous gas monitor (MultiRAE). The total amount of ammonia was compared by summing the amount of gas measured twice for each treatment group.

As a result, as shown in FIG. 5, the total generation of ammonia gas was reduced by 41%, as compared with a non-CJBS303 strain-added control, indicating that the reducing effect was 4 times or higher, as compared with the standard strain.

(2) Effect of reducing manure-derived odorous gases

A probiotic containing the CJBS303 strain was fed to pigs, and its effect on generation of manure-derived odorous gas was examined. A total of 32 heads of finishing pigs were divided into two groups, and a feeding experiment was performed. A group fed with an additive-free feed was set as a control, and the control feed was fed to finishing pigs of a total of 4 pens, four heads of finishing pigs each pen, for 5 weeks. Meanwhile, a group that fed with a feed containing $1x10^9$ cfu/kg of the CJBS303 strain in the feed was set as a treatment group, and the feed was fed to finishing pigs of a total of 4 pens, four heads of finishing pigs each pen, for 5 weeks. Manures derived from the control and treatment groups were sampled every week from week 2 after feeding, and the odorous gases generated therefrom were measured. The manures sampled from the control and treatment groups were mixed at a constant ratio (600 g of feces + 1.2 L of 12.5% diluted urine), and then subdivided into 300 ml in a bottle, followed by stationary incubation at 39°C under facultative aerobic conditions for a total of 42 hr. 12 hr, 18 hr, 36 hr, and 42 hr from the time of incubation, the amounts of generated gases, i.e., hydrogen sulfide and ammonia gas, were measured for a total of four times using a portable odorous gas monitor (MultiRAE). The manure culture experiment was conducted every week when sampling of manure was performed, and a reduction rate relative to the control was calculated by averaging the data until week 5.

As a result, as shown in Table 2 and FIG. 6, the treatment group was observed to have lower concentrations of ammonia and hydrogen sulfide gases than the control from week 2 after feeding, and it was confirmed that when the concentrations of the gases generated until week 5 were averaged and compared, the reduction rate of odorous gases according to feeding of the CJBS303 strain was 38.3% for ammonia and 88.9% for hydrogen sulfide.

[Table 2]

| | Ammonia (ppm) | | Hydrogen sulfide (ppm) | |
|---|---|---|---|---|
| | Control | CJBS303 0.1% | Control | CJBS303 0.1% |
| Week 2 | 207 | 140 | 286 | 14 |
| Week 3 | 278 | 152 | 166 | 22 |
| Week 4 | 212 | 135 | 52 | 20 |
| Week 5 | 180 | 113 | 91 | 10 |
| Average | 219 | 135 | 149 | 16 |
| Reduction | | 38.30% | | 88.90% |
| rate relative to control | | | | |

(3) Effect of reducing odorous gases in pig pen

A probiotic containing the CJBS303 strain was fed to pigs, and its effect on generation of odorous gases in a pig pen was examined. A total of 96 heads of 74-day-old finishing pigs were divided into two groups, and a feeding experiment was performed. A group fed with an additive-free feed was set as a control, and the control feed was fed to growing pigs of a total of 12 pens, four heads of growing pigs each pen, for 4 weeks. Meanwhile, a group that fed with a feed containing $1x10^9$ cfu/kg of the CJBS303 strain in the feed was set as a treatment group, and the feed was fed to growing pigs of a total of 12 pens, four heads of growing pigs each pen, for 4 weeks. The control and treatment groups were respectively bred in independent pig pens, and to exclude the effects of the existing slurries in the pig pens, the slurries of the pig pens for the control and treatment groups were removed at the same time, before start of the experiment. From 4 days before start of the experiment, gases in the pig pens were measured with respect to the pig pens of the control and treatment groups at 9 am and 4 pm daily. Ammonia, hydrogen sulfide, complex odor, and volatile fatty acids (VOCs) were measured using a precision odor measurement device (Odor Catch, SLC-OP-1350, Scientec Lab Center Co., LTD), and methyl mercaptan was measured using a portable odorous gas monitor (MultiRAE). The daily measured data were used to calculate average values thereof for each week.

As a result, as shown in Table 3 and FIGS. 7A to 7E, the effect of reducing the odorous gases according to feeding of the CJBS303 strain was observed in all measurement items, and this effect was more pronounced at week 4 after each feeding. In particular, in the treatment group, hydrogen sulfide and methyl mercaptan which are sulfur-based odorous gases showed a reduction rate of 50% or more (Duncan, $p>0.01$), a complex odor showed a reduction rate of 20% or more (Duncan, $p>0.05$), and ammonia and volatile fatty acids showed a reduction rate of 17% and 20%, respectively.

[Table 3]

| | Complex odor (OU) | Ammonia (ppm) | Hydrogen sulfide (ppm) | VOCs (ppm) | Methyl mercaptan (ppm) |
|---|---|---|---|---|---|
| Control | $591^{a}$ | 7.7 | $0.88^{a}$ | 2.13 | $1.6^{a}$ |
| CJBS303 | $456^{b}$ | 6.4 | $0.41^{b}$ | 1.7 | $0.6^{b}$ |
| Reduction rate (%) | 22.80% | 17.40% | 53.70% | 20.10% | 60.30% |

Example 4. Evaluation of Pig Productivity according to Feeding of CJBS303 strain

A probiotic containing the CJBS303 strain was fed to pigs, and its effect on pig productivity was examined. Under the same conditions as in (3) of Example 3, a total of 96 heads of 74-day-old finishing pigs were divided into two groups, and a feeding experiment was performed. The average daily gain (ADG) was calculated by measuring the body weight of the individual before start of the feeding experiment and 4 week after the end of the experiment, and the average daily feed intake (ADFI) and feed conversion ratio (FCR) were calculated by measuring the daily feeding amount and balance.

As a result, as shown in Table 4, in the treatment group fed with the CJBS303 strain, ADFI was improved by 7.3% (Duncan, $p<0.1$), and ADG was improved by 9.0% (Duncan, $p<0.03$). FCR was also improved by about 0.03. These experimental results indicate that feeding of the CJBS303 strain not only reduces the amount of odorous gases, but also exhibits a positive effect on pig productivity.

[Table 4]

| | | Control | CJBS303 |
|---|---|---|---|
| BW (kg) | Start | 38.69 | 38.66 |
| | Week 4 | 63.77 | 66.01 |
| ADG(kg) | | $0.90^{b}$ | $0.98^{a}$ |
| ADFI(kg) | | $2.35^{b}$ | $2.52^{a}$ |
| FCR | | 2.62 | 2.59 |

Example 5. Verification of Effect of Reducing Odorous Gases in Pig Pen according to Feeding of CJBS303 strain

To confirm reproducibility of the effect of reducing odorous gases in a pig pen according to feeding of the CJBS303 strain, a feeding test was conducted on two pig farms. A first farmhouse (A) was selected as a growing pig pen (A-1) and a finishing pig pen (A-2), and a second farmhouse (B) was selected as a finishing pig pen, and the test subjects and the number of individuals for each farm are as in Table 5.

[Table 5]

| | A-1 | A-2 | B |
|---|---|---|---|
| Test subject | Growing pig | Finishing pig | Finishing pig |
| Number of individuals | 650 heads | 800 heads | 944 heads |

Growing pigs or finishing pigs in both farms were fed with a test feed containing $2x10^9$ cfu/kg of the CJBS303 strain for 3 weeks, and the reduction rate was calculated by measuring the concentrations of odorous gases in the pig pens before feeding of the test feed and week 3 after feeding. Ammonia, hydrogen sulfide, and methyl mercaptan were measured using a portable odorous gas monitor (MultiRAE), and complex odor was measured using a precision odor measurement device (Odor Catch, SLC-OP-1350, Scientec Lab Center Co., LTD) at 9 am.

As a result, as shown in FIGS. 8A to 8D, in the treatment group fed with the CJBS303 strain, ammonia showed a reduction rate of 39% or more, hydrogen sulfide showed a reduction rate of 26% or more, methyl mercaptan showed a reduction rate of 24% or more, and complex odor showed a reduction rate of 21% or more. In addition, the effect of reducing the odorous gases was observed from week 3 after feeding, regardless of the growing stage or the finishing stage which are pig growth stages.

Although the above description of the present disclosure has been described for illustrative purposes, those skilled in the art will appreciate that the present disclosure may be implemented in a different specific form without changing the technical spirit or essential characteristics thereof. Therefore, it should be understood that the above embodiments are not limitative, but illustrative in all aspects.

International Depositary Authority: Korean Culture Center of Microorganisms (foreign)

Accession No.: KCCM12435P
Date of deposit: 20190213

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT issued pursuant to Rule 7.1 by the INTERNATIONAL DEPOSITARY AUTHORITY identified at the bottom of this page

| I. IDENTIFICATION OF THE MICROORGANISM | |
|---|---|
| Identification reference given by the DEPOSITOR: *Bacillus subtilis* CJBS 303 | Accession number given by the INTERNATIONAL DEPOSITARY AUTHORITY: KCCM12435P |
| II. SCIENTIFIC DESCRIPTION AND/OR PROPOSED TAXONOMIC DESIGNATION | |
| The microorganism identified under I above was accompanied by:<br>☐ a scientific description<br>☐ a proposed taxonomic designation<br>(Mark with a cross where applicable) | |
| III. RECEIPT AND ACCEPTANCE | |
| This International Depositary Authority accepts the microorganism identified under I above, which was received by it on February. 13. 2019 (date of the original deposit).[1] | |
| IV. RECEIPT OF REQUEST FOR CONVERSION | |
| The microorganism identified under I above was received by this International Depositary Authority on (date of the original deposit) and a request to convert the original deposit to a deposit under the Budapest Treaty was received by it on (date of receipt of request for conversion). | |
| V. INTERNATIONAL DEPOSITARY AUTHORITY | |
| Name : Korean Culture Center of Microorganisms<br><br>Address : Yurim B/D<br>45, Hongjenae-2ga-gil<br>Seodaemun-gu<br>SEOUL 03641<br>Republic of Korea | Signature(s) of person(s) having the power to represent the International Depositary Authority or of authorized official(s):<br><br>Date: February. 13. 2019. |

[1] Where Rule 6.4(d) applies, such date is the date on which the status of international depositary authority was acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

**1.** A *Bacillus subtilis* CJBS303 strain deposited with the Accession No. KCCM12435P.

**2.** The *Bacillus subtilis* CJBS303 strain of claim 1, wherein the strain has an effect of reducing livestock manure-derived odors.

**3.** The *Bacillus subtilis* CJBS303 strain of claim 1, wherein the strain reduces levels of one or more odorous gases selected from the group consisting of ammonia, hydrogen sulfide, methyl mercaptan, indoles, phenols, and volatile fatty acids (VOCs).

**4.** A composition comprising the *Bacillus subtilis* CJBS303 strain of claim 1, a culture of the strain, a concentrate of the culture, or a dry product of the concentrate.

**5.** The composition of claim 4, further comprising a cryoprotectant or an excipient.

**6.** The composition of claim 5, wherein the cryoprotectant is one or more selected from the group consisting of glycerol, trehalose, maltodextrin, skimmed milk, and starch.

**7.** A feed composition comprising the composition of claim 4.

**8.** The feed composition of claim 7, comprising $1.0 \times 10^7$ cfu/kg to $1.0 \times 10^{11}$ cfu/kg of the *Bacillus subtilis* CJBS303 strain, based on the total weight of the composition.

**9.** A microbial preparation for removing livestock odors, comprising the *Bacillus subtilis* CJBS303 strain of claim 1.

**10.** The microbial preparation of claim 6, wherein the microbial preparation reduces levels of one or more odorous gases selected from the group consisting of ammonia, hydrogen sulfide, methyl mercaptan, indoles, phenols, and volatile fatty acids (VOCs).

**11.** A method of reducing odors from manure of an individual, comprising administering, to the individual, the strain, the composition, or the microbial preparation of any one of claims 1 to 10.

**12.** A method of preparing a microbial preparation, comprising:

culturing a *Bacillus subtilis* CJBS303 strain deposited with the Accession No. KCCM12435P; and
mixing the recovered strain with an additive.

**13.** The method of claim 12, wherein the additive is a cryoprotectant, and the method further comprises freeze-drying the obtained mixture, after the mixing.

**14.** The method of claim 13, wherein the strain in a freeze-dried microbial preparation obtained by the freeze-drying is in a viable state.

**15.** The method of claim 12, wherein the microbial preparation comprises $1.0 \times 10^7$ cfu/kg to $1.0 \times 10^{11}$ cfu/kg of the *Bacillus subtilis* CJBS303 strain, based on the total weight of the microbial preparation.

FIG. 1

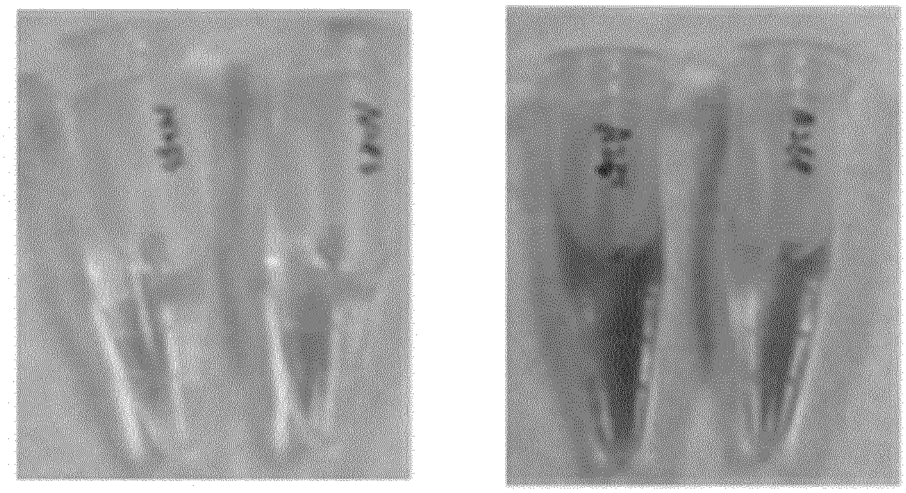

CONTROL CJBS303

FIG. 2

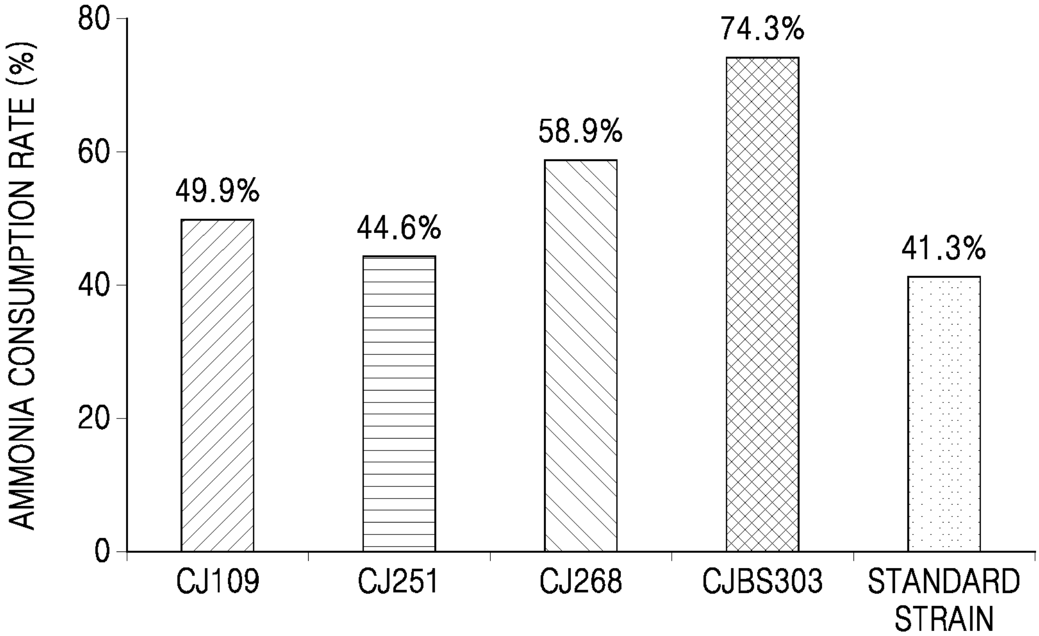
AMMONIA CONSUMPTION RATE (%)
80
60
40
20
0
49.9%
44.6%
58.9%
74.3%
41.3%
CJ109
CJ251
CJ268
CJBS303
STANDARD STRAIN

FIG. 3

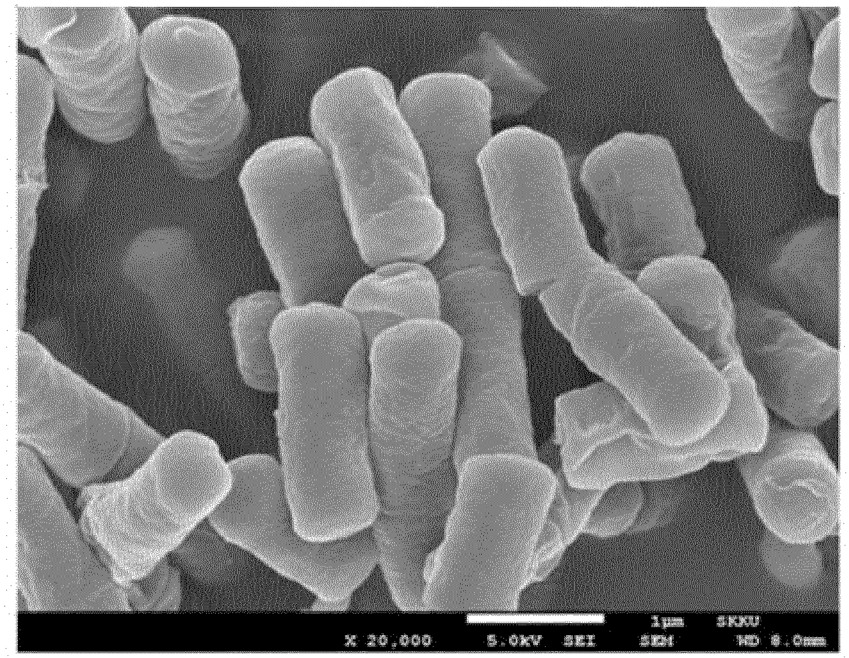

FIG. 4

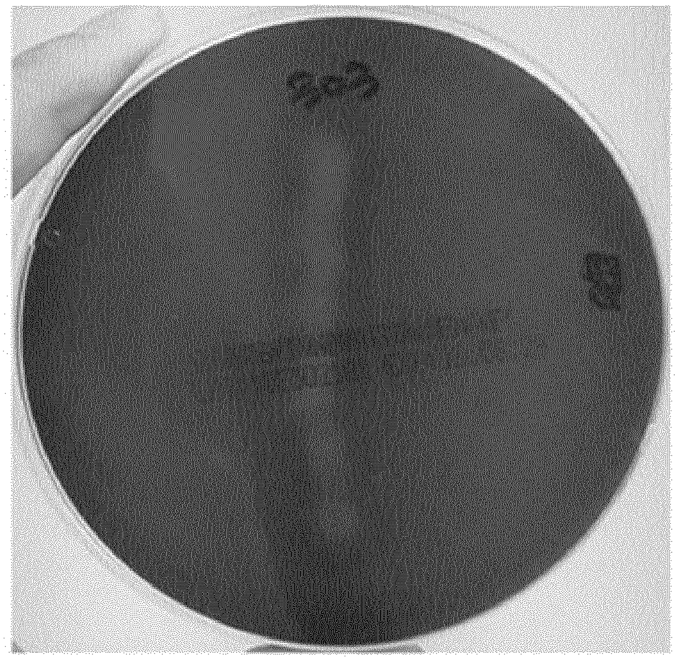

FIG. 5

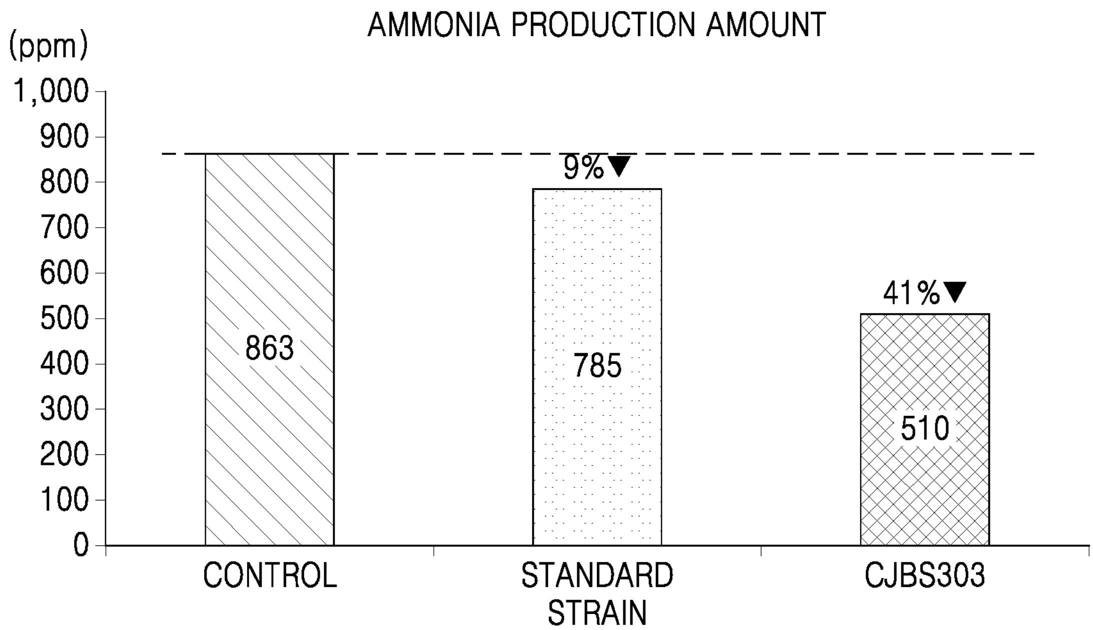
AMMONIA PRODUCTION AMOUNT
(ppm)
1,000
900
800
700
600
500
400
300
200
100
0
863
9%▼
785
41%▼
510
CONTROL
STANDARD STRAIN
CJBS303

FIG. 6

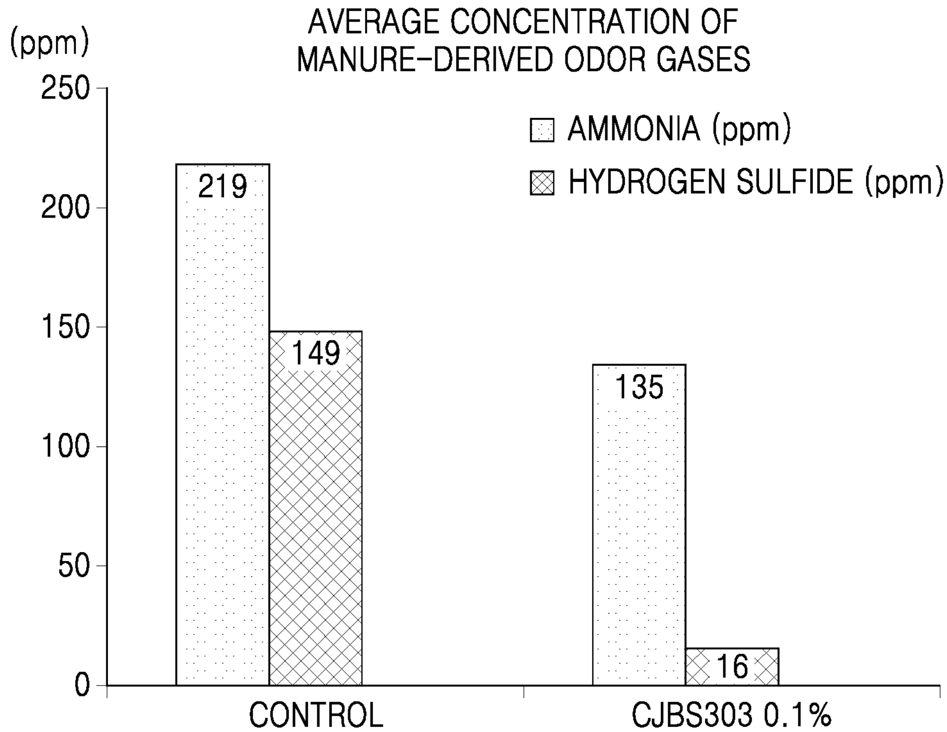
AVERAGE CONCENTRATION OF
MANURE-DERIVED ODOR GASES
(ppm)
250
200
150
100
50
0
AMMONIA (ppm)
HYDROGEN SULFIDE (ppm)
219
149
135
16
CONTROL
CJBS303 0.1%

FIG. 7A

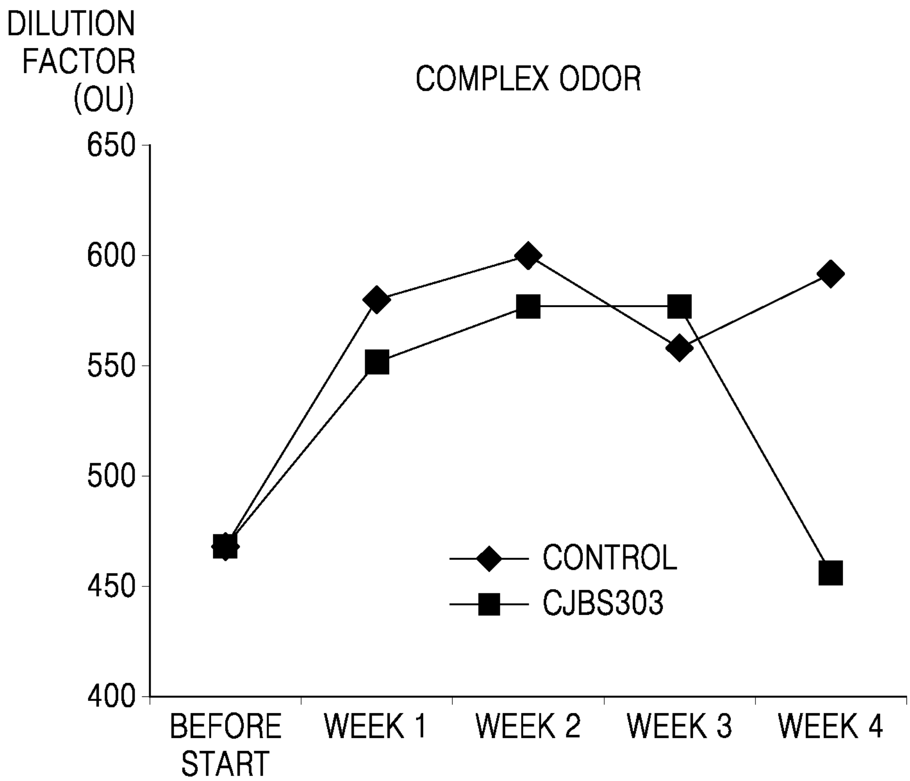
DILUTION
FACTOR
(OU)
COMPLEX ODOR
650
600
550
500
450
400
CONTROL
CJBS303
BEFORE
START
WEEK 1
WEEK 2
WEEK 3
WEEK 4

FIG. 7B

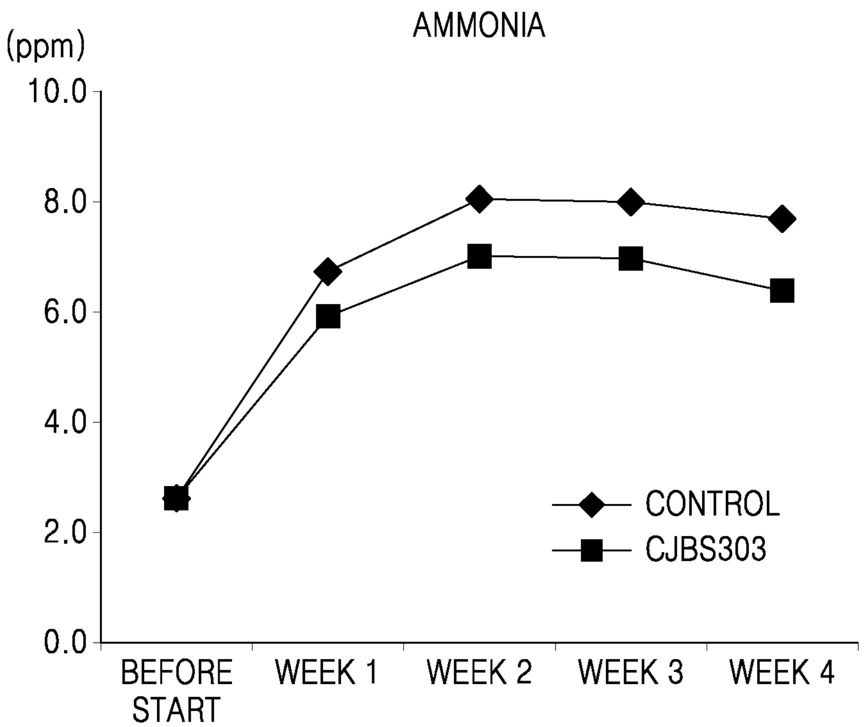
AMMONIA
(ppm)
10.0
8.0
6.0
4.0
2.0
0.0
BEFORE START
WEEK 1
WEEK 2
WEEK 3
WEEK 4
CONTROL
CJBS303

FIG. 7C

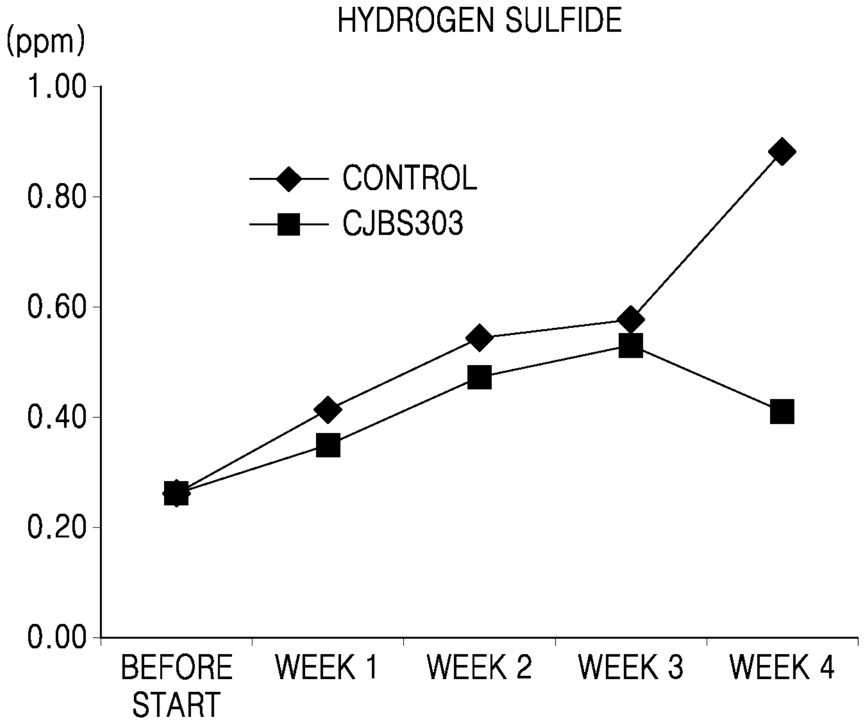
HYDROGEN SULFIDE
(ppm)
1.00
0.80
0.60
0.40
0.20
0.00
CONTROL
CJBS303
BEFORE START
WEEK 1
WEEK 2
WEEK 3
WEEK 4

FIG. 7D

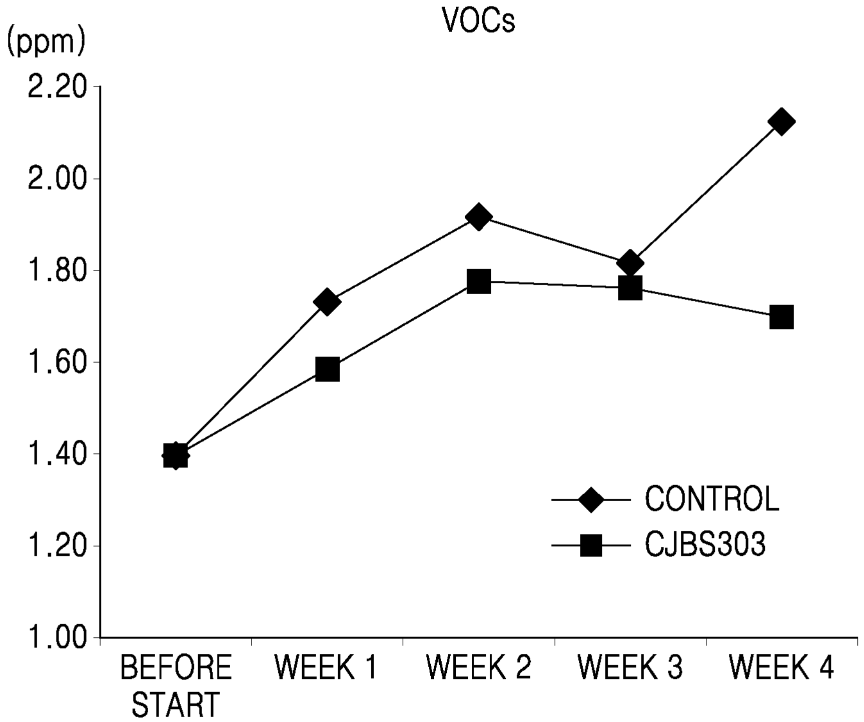
VOCs
(ppm)
2.20
2.00
1.80
1.60
1.40
1.20
1.00
BEFORE START
WEEK 1
WEEK 2
WEEK 3
WEEK 4
CONTROL
CJBS303

FIG. 7E

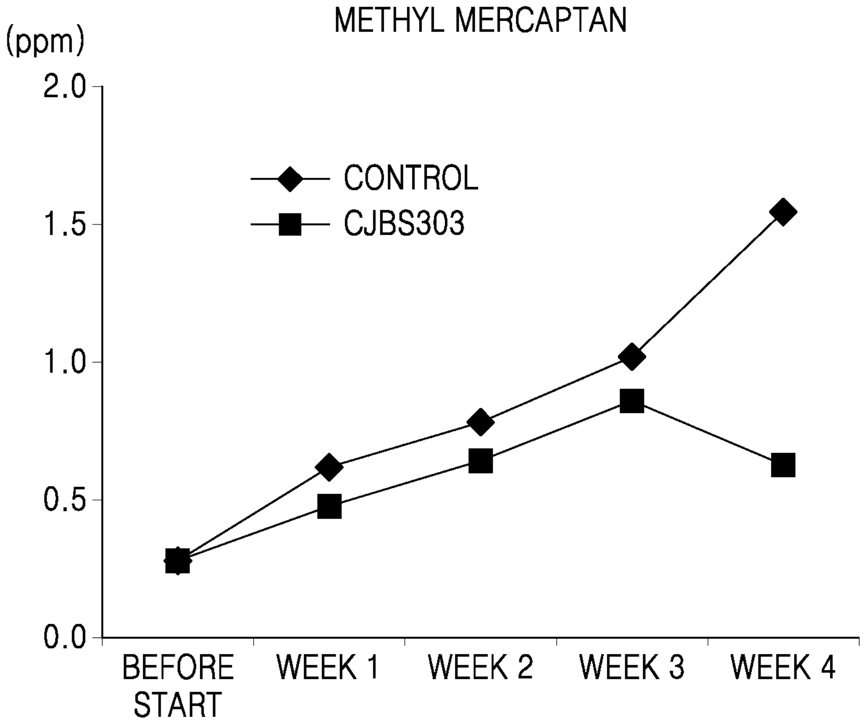
METHYL MERCAPTAN
(ppm)
2.0
1.5
1.0
0.5
0.0
CONTROL
CJBS303
BEFORE START
WEEK 1
WEEK 2
WEEK 3
WEEK 4

FIG. 8A

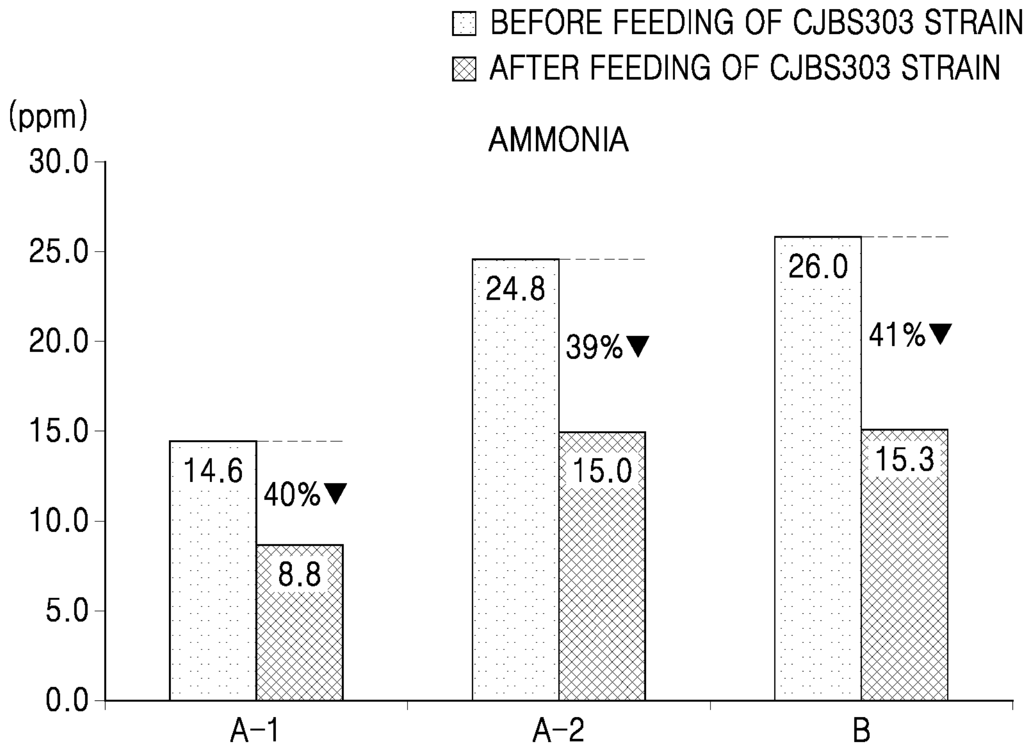
BEFORE FEEDING OF CJBS303 STRAIN
AFTER FEEDING OF CJBS303 STRAIN
(ppm)
AMMONIA
30.0
25.0
20.0
15.0
10.0
5.0
0.0
14.6
40%▼
8.8
24.8
39%▼
15.0
26.0
41%▼
15.3
A-1
A-2
B

FIG. 8B

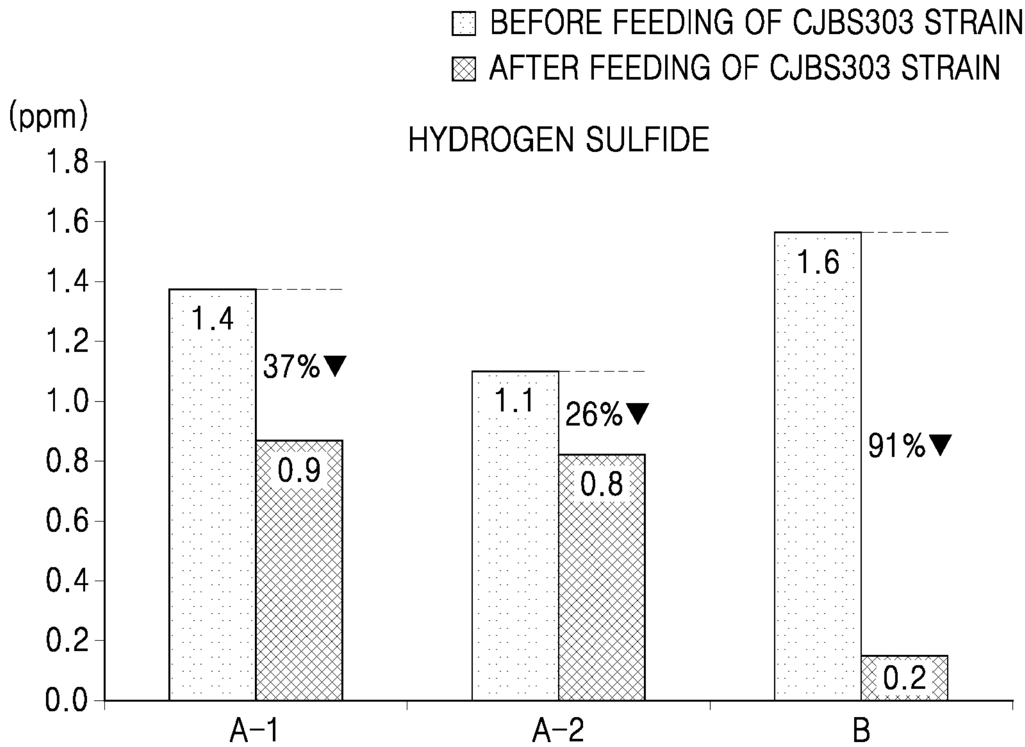
BEFORE FEEDING OF CJBS303 STRAIN
AFTER FEEDING OF CJBS303 STRAIN
(ppm)
HYDROGEN SULFIDE
1.8
1.6
1.4
1.2
1.0
0.8
0.6
0.4
0.2
0.0
1.4
37%▼
0.9
1.1
26%▼
0.8
1.6
91%▼
0.2
A-1
A-2
B

FIG. 8C

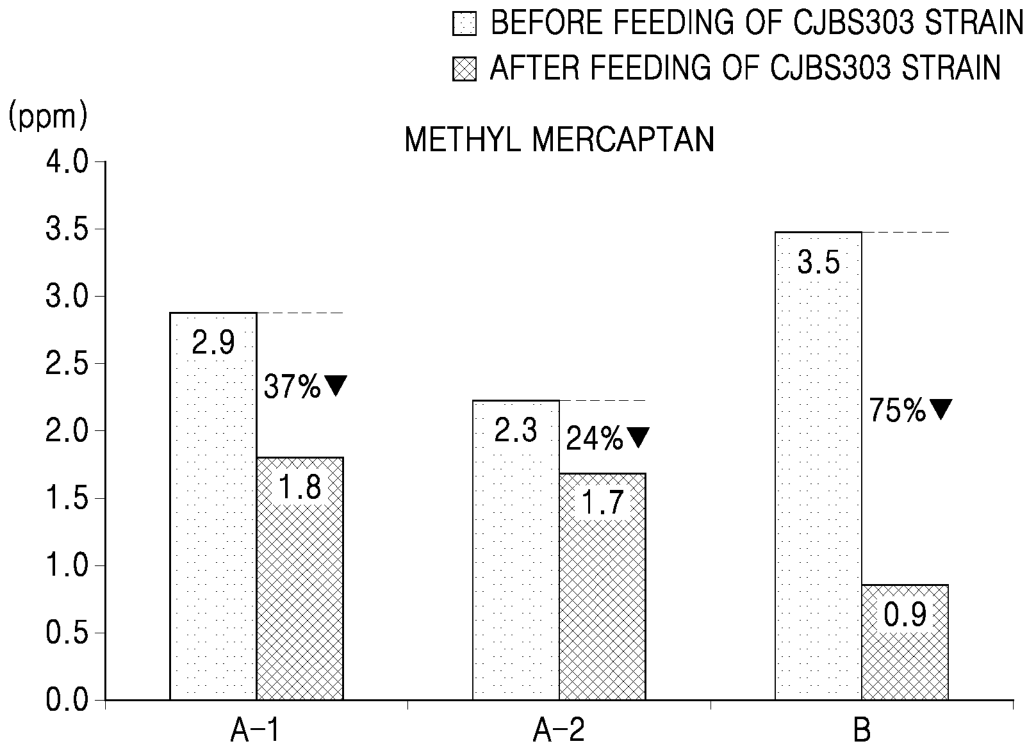
BEFORE FEEDING OF CJBS303 STRAIN
AFTER FEEDING OF CJBS303 STRAIN
(ppm)
METHYL MERCAPTAN
4.0
3.5
3.0
2.5
2.0
1.5
1.0
0.5
0.0
2.9
37%▼
1.8
2.3
24%▼
1.7
3.5
75%▼
0.9
A-1
A-2
B

FIG. 8D

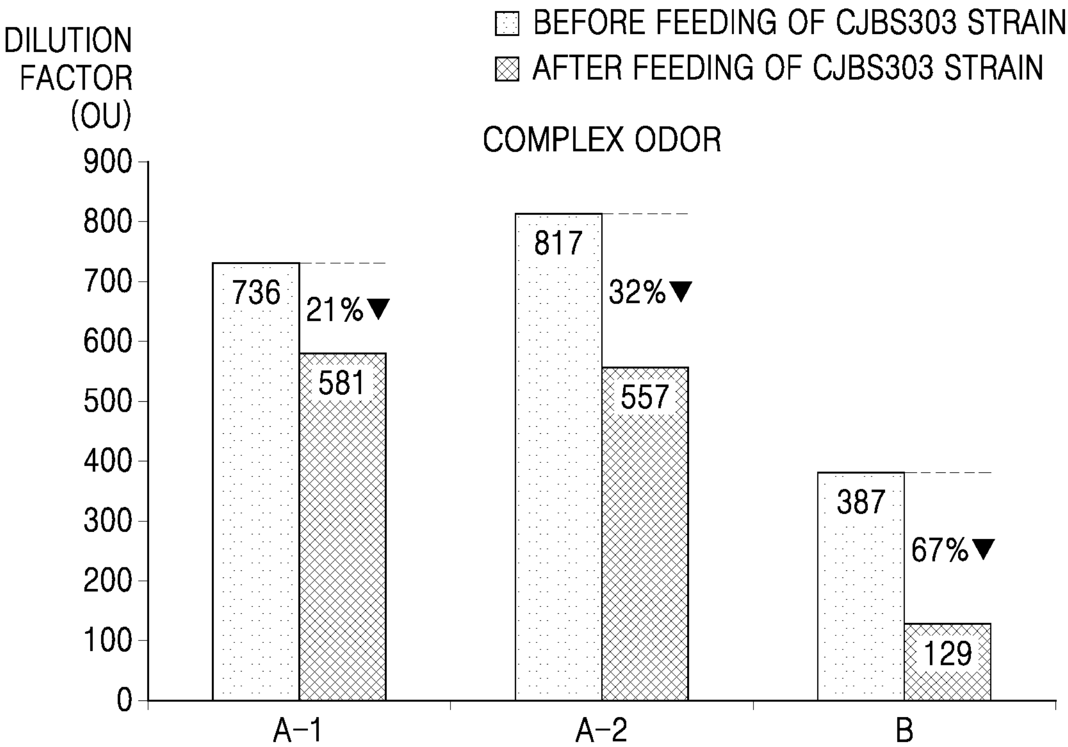
BEFORE FEEDING OF CJBS303 STRAIN
AFTER FEEDING OF CJBS303 STRAIN
DILUTION FACTOR (OU)
COMPLEX ODOR
900
800
700
600
500
400
300
200
100
0
736
21%▼
581
817
32%▼
557
387
67%▼
129
A-1
A-2
B

**INTERNATIONAL SEARCH REPORT**

International application No.
**PCT/KR2020/000133**

A. CLASSIFICATION OF SUBJECT MATTER

***C12N 1/20(2006.01)i, A23K 10/18(2016.01)i, B01D 53/84(2006.01)i, C12R 1/125(2006.01)n***

According to International Patent Classification (IPC) or to both national classification and IPC

B. FIELDS SEARCHED

Minimum documentation searched (classification system followed by classification symbols)
C12N 1/20; A01N 63/02; A23K 1/16; A61L 9/00; A61L 9/01; A61L 9/013; A23K 10/18; B01D 53/84; C12R 1/125

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: bacillus subtilis, odor, livestock excretions, feed, freeze drying

C. DOCUMENTS CONSIDERED TO BE RELEVANT

| Category* | Citation of document, with indication, where appropriate, of the relevant passages | Relevant to claim No. |
|---|---|---|
| X | KR 10-2017-0111309 A (MICROBIAL INSTITUTE FOR FERMENTATION INDUSTYRY) 12 October 2017<br>See claims 1, 4, 5; paragraphs [0004], [0021], [0022], [0025], [0057], [0058]; table 2. | 1-15 |
| X | US 2013-0064927 A1 (DAVIS, Mari Ellen et al.) 14 March 2013<br>See claims 1-14; paragraphs [0011], [0073]-[0075], [0118], [0161]. | 1-15 |
| A | KR 10-1260872 B1 (CHAMSHIN PHARMA CO., LTD.) 06 May 2013<br>See the entire document. | 1-15 |
| A | KR 10-0443156 B1 (ECOBIOGEN CO., LTD.) 05 August 2004<br>See the entire document. | 1-15 |
| A | KR 10-2012-0010017 A (REPUBLIC OF KOREA(MANAGEMENT : RURAL DEVELOPMENT ADMINISTRATION)) 02 February 2012<br>See the entire document. | 1-15 |

☐ Further documents are listed in the continuation of Box C.　☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 APRIL 2020 (13.04.2020) | **13 APRIL 2020 (13.04.2020)** |

Name and mailing address of the ISA/KR
Korean Intellectual Property Office
Government Complex Daejeon Building 4, 189, Cheongsa-ro, Seo-gu, Daejeon, 35208, Republic of Korea
Facsimile No. +82-42-481-8578

Authorized officer

Telephone No.

Form PCT/ISA/210 (second sheet) (January 2015)

**INTERNATIONAL SEARCH REPORT**
Information on patent family members

International application No.
**PCT/KR2020/000133**

| Patent document cited in search report | Publication date | Patent family member | Publication date |
|---|---|---|---|
| KR 10-2017-0111309 A | 12/10/2017 | CN 107384817 A | 24/11/2017 |
| | | KR 10-1808847 B1 | 13/12/2017 |
| US 2013-0064927 A1 | 14/03/2013 | BR 112014003950 A2 | 13/06/2017 |
| | | CA 2845576 A1 | 28/02/2013 |
| | | CN 103930540 A | 16/07/2014 |
| | | CN 103930540 B | 17/05/2017 |
| | | CN 107418908 A | 01/12/2017 |
| | | DK 2748300 T3 | 14/01/2019 |
| | | EP 2748300 A1 | 02/07/2014 |
| | | EP 2748300 B1 | 19/09/2018 |
| | | ES 2702230 T3 | 28/02/2019 |
| | | PL 2748300 T3 | 31/05/2019 |
| | | RU 2014119583 A | 20/11/2015 |
| | | US 10058110 B2 | 28/08/2018 |
| | | US 2015-0230498 A1 | 20/08/2015 |
| | | US 9089151 B2 | 28/07/2015 |
| | | WO 2013-029013 A1 | 28/02/2013 |
| | | WO 2013-029013 A8 | 28/09/2017 |
| KR 10-1260872 B1 | 06/05/2013 | US 2011-0297757 A1 | 08/12/2011 |
| | | US 8413681 B2 | 09/04/2013 |
| KR 10-0443156 B1 | 05/08/2004 | None | |
| KR 10-2012-0010017 A | 02/02/2012 | KR 10-1190650 B1 | 17/10/2012 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 101229865
- KR 1020120088436